# EUROPEAN PATENT APPLICATION

(11) **EP 4 403 163 A1**
(43) Date of publication of application: **24.07.2024**
(21) Application number: 22870162.9
(22) Date of filing: 24.08.2022
(51) Int. Cl.: A61K 8/49, A61Q 19/08, A61K 31/352, A61P 17/00, C07D 311/30

(54) **ALTERNATIVE NOVEL MATERIAL TO BOTULINUM NEUROTOXIN AND PREPARATION METHOD THEREFOR**

(30) Priority: 17.09.2021 KR 20210124504
(71) Applicant: Intron Biotechnology, Inc., Seongnam-si, Gyeonggi-do 13202 (KR)
(72) Inventor: YOON, Seong Jun, Seoul 06281 (KR); KWON, An Sung, Gwangju-Si Gyeonggi-do 12734 (KR); JUN, Soo Youn, Seoul 06518 (KR); PARK, Ji Sung, Seongnam-si Gyeonggi-do 13464 (KR); PARK, Seon Ho, Seoul 05101 (KR); KANG, Sang Hyeon, Seoul 05501 (KR)
(74) Representative: Regimbeau
(86) International application number: PCT/KR2022/012651
(87) International publication number: WO 2023/043076

(57) **Abstract**

The present invention relates to an alternative novel material to botulinum neurotoxin and a preparation method thereof, and more specifically, the present invention relates to a myricetin acetyl derivative and a preparation method thereof, wherein the myricetin acetyl derivative has significantly improved stability, including in-vivo stability, and has the ability to inhibit SNARE complex formation, and as a result, to inhibit acetylcholine release.

## Description

### Technical Field

The present disclosure relates to a novel material alternative to botulinum neurotoxin and a method of preparing the same. More specifically the present disclosure relates to myricetin acetyl derivative having a dramatically improved stability including in-vivo stability as well as an ability to inhibit the release of acetylcholine and to a method of preparing the myricetin acetyl derivative.

### Background Art

Botulinum neurotoxin cleaves soluble N-ethylmaleimide-sensitive factor attachment protein receptor (SNARE) proteins mediating the docking of the presynaptic membrane and synaptic vesicles in nerve cells, resulting in inhibition of the release of acetylcholine, and ultimately blocking nerve transmission. Thus, botulinum neurotoxin can be used as a material for the treatment of over 100 disorders caused by excessive release of acetylcholine, such as crossed eyes and excessive sweating, as well as aesthetic applications such as treating wrinkles.

Botulinum neurotoxin itself is a toxin so botulinum neurotoxin is known to not only cause death when administered incorrectly, but also to cause side effects such as allergic reactions, slurred speech, awkward facial expressions, and inability to close eyes. Generally, the side effects are caused by failure to control the amount of botulinum neurotoxin used and the phenomenon of botulinum neurotoxin spreading to unwanted muscles. These side effects can be resolved only when the botulinum neurotoxin is broken down in the body and new nerves are formed to restore normal function, which may take several months. For this reason, a considerable level of caution is required when using botulinum neurotoxin for cosmetic or medical purposes. Botulinum neurotoxin is produced by culturing an anaerobic microorganism called *Clostridium botulinum.* The bacterium is very fatal. Therefore, the entire production process is required to be strictly managed, from the culturing process to separation, purification, disposal, and sterilization.

For this reason, attempts are being made to develop various materials that can be alternative to botulinum neurotoxin. The attempts have been made by using proteins or polypeptides (Korean Patent Application Publication No. 10-2012-0001964 and Korean Patent Application Publication No. 10-2012-0122999), by using natural polyphenols (Korean Patent Application Publication No. 10-2008-0083438, Korean Patent Application Publication No. 10-2008-0091735, Korean Patent Application Publication No. 10-2011-0017599, and Korean Patent Application Publication No. 10-2016-0058739), and by using myricetin derivatives (Korean Patent Application Publication No. 10-2017-0015845 and Korean Patent Application Publication No. 10-2017-0091554). Among these materials, peptide has been commercialized as a botulinum neurotoxin alternative overseas. Lipotec's Argireline is one example. However, the commercial success of botulinum neurotoxin alternatives is still minimal due to several shortcomings.

Accordingly, there is a need for the development of novel materials that can overcome the shortcomings of existing materials alternative to botulinum neurotoxin while also solving the side effects and problems of botulinum neurotoxin.

### Disclosure

### Technical Problem

Accordingly, the present inventors focused on the fact that among the existing alternative materials to botulinum neurotoxin, myricetin derivatives have a decreased stability including decreased in-vivo stability, and focused on the fact that by solving this problem, more valuable materials alternative to botulinum neurotoxin can be developed. Given the facts, the present inventors made diligent efforts to solve the instability problem of myricetin derivatives, and as a result, the present inventors completed the present disclosure by developing a material with dramatically improved stability compared to previously disclosed myricetin derivatives and also by developing a method of preparing the material.

Therefore, one objective of the present disclosure is to provide a myricetin derivative (3-acetyl-myricetin) represented by Formula 1, having a dramatically improved stability that can be used as a material alternative to botulinum neurotoxin as well as having the ability to inhibit SNARE complex formation and consequently inhibit acetylcholine release.

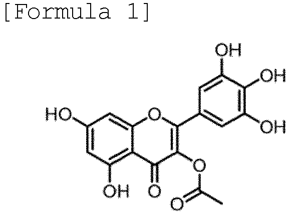

Another objective of the present disclosure is to provide a method of preparing a myricetin acetyl derivative represented by [Formula 1].

Yet another objective of the present disclosure is to provide a cosmetic composition for aesthetic purposes used as an alternative to botulinum neurotoxin, the cosmetic composition containing the myricetin acetyl derivative represented by [Formula 1] and used as an active ingredient.

In addition, the other objective of the present disclosure is to provide a pharmaceutical composition for treatment purposes of disorders caused by excessive release of acetylcholine, the pharmaceutical composition including the myricetin acetyl derivative represented by [Formula 1] and used as an active ingredient.

### Technical Solution

Therefore, in one aspect of the present disclosure, the present disclosure provides a myricetin acetyl derivative represented by [Formula 1] and having the ability to inhibit SNARE complex formation and consequently inhibit acetylcholine release.

Of course, it is obvious within the present disclosure that the effects of the present disclosure can be obtained even when the myricetin acetyl derivative has a skeleton represented by [Formula 1] as its basic skeleton and is additionally derivatized.

In another aspect of the present disclosure, the present disclosure provides a cosmetic composition for aesthetic purposes containing a myricetin acetyl derivative represented by [Formula 1] and used as an active ingredient.

The aesthetic purposes may include improving wrinkles, reducing pore size, and improving skin elasticity, but are naturally not limited thereto. The wrinkles may include brow droop, crow's-feet, nasolabial folds, mouth frowns, neck wrinkles, forehead lines, frown lines, tear troughs, and jowls, but are not limited thereto.

In yet another aspect of the present disclosure, "cosmetic composition" is a concept that includes all compositions that beautify the appearance of the human body. The cosmetic composition for aesthetic purposes of the present disclosure refers to a composition that beautifies the appearance of the human body by suppressing adverse effects on the skin as a physiological phenomenon according to biological rhythm, but the cosmetic composition is not limited to the concept.

In yet another aspect of the present disclosure, since the cosmetic composition is basically applied to the skin, the cosmetic composition can be prepared in any commonly prepared formulation with reference to cosmetic compositions in the art. For example, the formulation of the cosmetic composition may include a solution, suspension, emulsion, paste, gel, cream, lotion, powder, oil, powder foundation, emulsion foundation, wax foundation, and spray, but is not limited thereto. More specifically, the formulation of the cosmetic composition may include flexible lotion, nourishing lotion, nourishing cream, massage cream, essence, eye cream, spray, or powder.

In yet another aspect of the present disclosure, when the formulation of the present disclosure is a paste, cream, or gel, the present disclosure may include animal oil, vegetable oil, wax, paraffin, starch, tragacanth, cellulose derivatives, polyethylene glycol, silicone, bentonite, silica, talc, and zinc oxide as carrier ingredients.

In yet another aspect of the present disclosure, when the formulation of the present disclosure is a powder or spray, the present disclosure may include lactose, talc, silica, aluminum hydroxide, calcium silicate, and polyamide powder as carrier ingredients. Particularly, when the formation is a spray, the present disclosure may additionally include propellants such as chlorofluorohydrocarbon, propane/butane, and dimethyl ether.

In yet another aspect of the present disclosure, when the formulation of the present disclosure is a solution or emulsion, the present disclosure may include a solvent, solubilizing agent, and emulsifying agent as carrier ingredients. The present disclosure specifically includes water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, and fatty acid ester of sorbitan.

In yet another aspect of the present disclosure, when the dosage form of the present disclosure is a suspension, the present disclosure may include liquid diluents such as water, ethanol, and propylene glycol as carrier ingredients; suspension agents such as ethoxylated isostearyl alcohol, polyoxyethylene sorbitol ester, and polyoxyethylene sorbitan ester; and microcrystalline cellulose, aluminum metahydroxide, bentonite, agar, and tragacanth.

In yet another aspect of the present disclosure, when the formulation of the present disclosure is a surfactant-containing cleansing, as carrier ingredients, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, lanolin derivative, or ethoxylated glycerol fatty acid ester can be used.

In yet another aspect of the present disclosure, the present disclosure provides a pharmaceutical composition for treatment purposes of diseases caused by the excessive release of acetylcholine, the pharmaceutical composition including a myricetin acetyl derivative represented by [Formula 1] and used as an active ingredient.

The diseases caused by the excessive release of acetylcholine include crossed eyes, inner ear disorder, teeth grinding, keloid scarring, hyperhidrosis, back pain, tremors, anal fissure, buttock deformity, muscle injuries (twitching), chronic migraine, depression, facial nerve disorder, neck pain (spasms), thyroid disorder, cardiac muscle disorder, upper limb spasticity, pancreas disorders, irritable bowel syndrome, stretch marks, and juvenile cerebral palsy, but are not limited thereto.

The pharmaceutically acceptable carrier contained in the pharmaceutical composition of the present disclosure is commonly used in preparation. The carrier may include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, but is not limited thereto. In addition to the ingredients, the pharmaceutical composition of the present disclosure may further include lubricants, wetting agents, sweeteners, flavoring agents, emulsifiers, suspending agents, and preservatives.

The pharmaceutical composition containing the myricetin acetyl derivative represented by [Formula 1] of the present disclosure and used as an active ingredient can be administered through oral or parenteral administration. Methods for parenteral administration may include intravenous administration, intraperitoneal administration, intramuscular administration, subcutaneous administration, intranasal administration, or local administration, but are not limited thereto.

The pharmaceutical composition containing the myricetin acetyl derivative represented by [Formula 1] of the present disclosure and used as an active ingredient is formulated using a pharmaceutically acceptable carrier and/or excipient according to a method that can be easily carried out by those skilled in the art to which the present disclosure pertains. The pharmaceutical composition may be prepared in unit dosage form or may be prepared by placing them in multi-dose containers. At this time, the formulation of the pharmaceutical composition may be a solution, suspension, or emulsion in an oil or aqueous medium, or may be an extract, powder, granule, tablet, or capsule, and the pharmaceutical composition even may further include a dispersant or stabilizer.

In addition, suitable application, spraying, and dosage of the pharmaceutical composition depend on factors such as formulation method, administration mode, age, body weight, sex, severity of disease symptoms, food, administration time, administration route, excretion rate, and reaction sensitivity. Generally, a skilled physician can easily determine and prescribe an effective dosage for the desired treatment.

According to yet another aspect of the present disclosure, the present disclosure provides a method of preparing a myricetin acetyl derivative represented by [Formula 1].

Specifically, the method of preparing the myricetin acetyl derivative represented by [Formula 1] is provided, the method including:
(A) inducing a reaction by adding acetic anhydride to myricetin dissolved in pyridine anhydrous under a nitrogen atmosphere sealed to prevent external air and moisture from being introduced;
(B) extracting with ethyl acetate (EA);
(C) vacuum-drying the ethyl acetate (EA) extract;
(D) diluting by adding dimethyl sulfoxide (DMSO) to the vacuum-dried sample and then adding phosphate-buffered saline (PBS; pH 7.0);
(E) incubating the diluted sample to decompose byproducts; and
(F) freeze-drying the incubated sample.

### Advantageous Effects

A myricetin acetyl derivative represented by [Formula 1] according to the present disclosure can provide improved stability. A composition containing the myricetin acetyl derivative used as an active ingredient is easier to store and handle, and more importantly, the composition can provide high in-vivo stability after administration, providing further improved and sustained efficacy.

### Description of Drawings

FIG. 1 shows a schematic diagram of the synthesis process of 3-acetyl-myricetin represented by [Formula 1] of the present disclosure;
FIG. 2 shows the results of reversed phase-high performance liquid chromatography (RP-HPLC) analysis of a freeze-dried sample in the production process of 3-acetyl-myricetin represented by [Formula 1] of the present disclosure;
FIG. 3 shows the results of RP-HPLC analysis of the final purified 3-acetyl-myricetin represented by [Formula 1];
FIG. 4 shows the results of liquid chromatography-mass spectrometry (LC/MS) analysis of the final purified 3-acetyl-myricetin represented by [Formula 1];
FIG. 5 shows the results of nuclear magnetic resonance (NMR) analysis of the final purified 3-acetyl-myricetin represented by [Formula 1];
FIG. 6 shows the results of measuring the degree of inhibition of acetylcholine release by 3-acetyl-myricetin represented by [Formula 1] according to the present disclosure, as shown in FIG. 6, 1 is the result of control, 2 is the result of the case with applying the 0.5 µM sample, 3 is the result of the case with applying the 2 µM sample, 4 is the result of the case with applying the 10 µM sample, and 5 is the result of the case with applying the 20 µM sample;
FIG. 7 shows the results of a stability comparison experiment of 3-acetyl-myricetin, myricetin, and myricetin-C4 represented by [Formula 1] according to the present disclosure; and

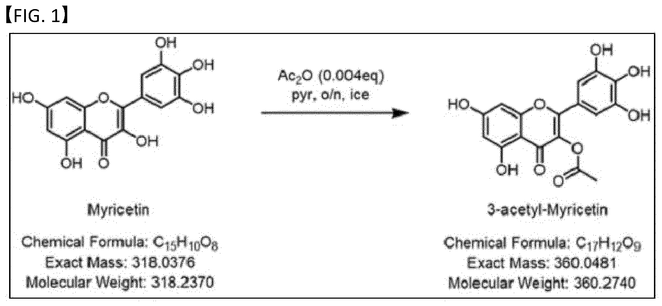

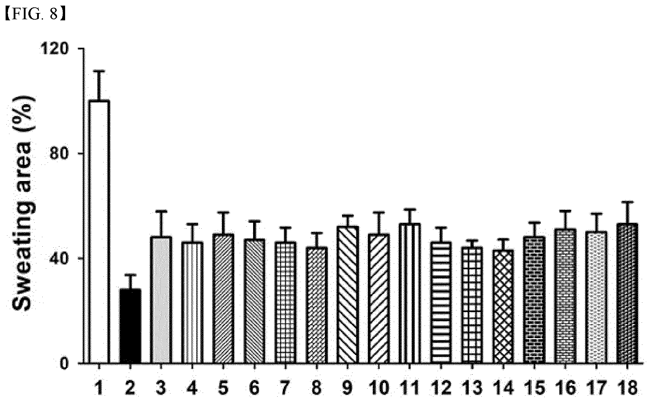

FIG. 8 shows the results of an efficacy comparison experiment, as shown in FIG. 8, 1 is the result of the control group (PBS-treated group; untreated group), 2 is the result of the 3-acetyl-myricetin-treated group, 3 is the result of the myricetin-treated group, 4 is the result of the laricitrin-treated group, 5 is the result of the combretol-treated group, 6 is the result of the syringetin-treated group, 7 is the result of the myricetin-C4-treated group, 8 is the result of the myricetin-C8-treated group, 9 is the result of the myricetin-C12-treated group, 10 is the result of the myricetin-C16-treated group, 11 is the result of the myricetin-C20-treated group, 12 is the result of the derivative E4-2-treated group, 13 is the result of the derivative E4-4-treated group, 14 is the result of the derivative E4-8-treated group, 15 is the result of the derivative E4-12-treated group, 16 is the result of the derivative E4-16-treated group, 17 is the result of the derivative E4-18-treated group, 18 is the result of the derivative E4-20-treated group. In the efficacy comparison experiment, the sweating area of the control group (untreated group) was set as 100% and the relative value was presented as the result.

### Mode for Disclosure

Hereinafter, the present disclosure will be described in more detail based on examples, but these examples are only illustrative of the present disclosure and the scope of the present disclosure is not limited to these examples.

### Example 1: Preparation of comparative substances and myricetin acetyl derivative

In this example, the comparative substances to be used in subsequent comparative experiments were prepared. The comparative substanes were selected as the followings: myricetin and myricetin derivatives described in Korean Patent Application Publication No. 10-2017-0015845 or Korean Patent Application Publication No. 10-2017-0091554. The myricetin derivatives are laricitrin of [Formula 2], combretol of [Formula 3], syringetin of [Formula 4], myricetin-C4 (which was prepared by lipase-mediated acylation using vinyl butyrate), myricetin-C8 (which was prepared by lipase-mediated acylation using vinyl octanoate), myricetin-C12 (which was prepared by lipase-mediated acylation using vinyl laurate), myricetin-C16 (which was prepared by lipase-mediated acylation using vinyl palmitate), myricetin-C20 (which was prepared by lipase-mediated acylation using vinyl eicosanoate), Derivative E4-2 (which was prepared by acylation using base, oxalyl chloride, and acetic acid), Derivative E4-4 (which was prepared by acylation using base, oxalyl chloride, and butyric acid), Derivative E4-8 (which was prepared by acylation using base, oxalyl chloride, and octanoic acid), Derivative E4-12 (which was prepared by acylation using base, oxalyl chloride, and lauric acid), Derivative E4-16 (which was prepared by acylation using base, oxalyl chloride, and palmitic acid), Derivative E4-18 (which was prepared by acylation using base, oxalyl chloride, and stearic acid), and Derivative E4-20 (which was prepared by acylation using base, oxalyl chloride, and arachidic acid).

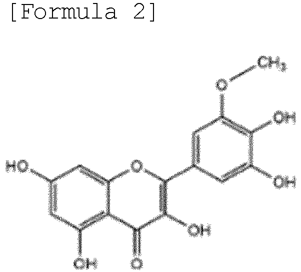

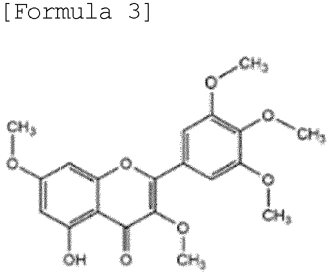

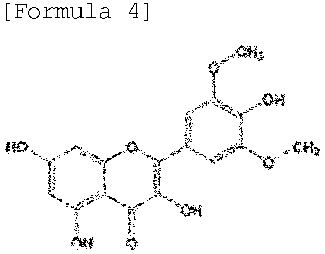

Myricetin, laricitrin, combretol, and syringetin commercially available were purchased. The remaining derivatives to be used as comparative substances were prepared according to the methods presented in the examples of Korean Patent Application Publication No. 10-2017-0015845 or Korean Patent Application Publication No. 10-2017-0091554. A myricetin acetyl derivative (i.e., 3-acetyl-myricetin) represented by [Formula 1] developed by the present inventors was prepared according to the following method. A stirring bar and myricetin (1 equivalent weight) were placed into a round bottom flask (RBF), and the flask was subjected to nitrogen purging. Next, the flask was closed with a rubber stopper to prevent the entering of external air and moisture, and then pyridine anhydrous (0.5 equivalent weight) was added to the RBF, and then all myricetin was dissolved in an ice bath. Acetic anhydride was added to the dissolved myricetin in a dropwise manner using a syringe. Stirring was performed overnight in the ice bath. After overnight reaction, the reaction was terminated by adding cold distilled water to the solution. After the reaction was terminated, all reaction solution was transferred to a separating funnel, and then ethyl acetate (EA) was added. EA was added to the residual solution in the RBF and the mixture solution was transferred to the separating funnel so that all reaction solution was recoverd. The separating funnel was shaken several times to ensure that the synthesized 3-acetyl-myricetin was well dissolved in EA, and then placed on a stand for incubation to separate water and EA phases. When the water and EA phases were separated, only the water phase was removed (The density of EA is 0.9, which is lower than that of water, so the water phase was placed below). After adding saturated brine (Brine) to the separating funnel, the separating funnel was shaken several times so that the synthesized 3-acetyl-myricetin could be well dissolved in EA, and then placed on a stand for incubation to separate water and EA phases. When the water and EA phases were separated, only the water phase was removed. The processes were repeated three times, the processes including, in short: shaking a separating funnel several times so that the synthesized 3-acetyl-myricetin could be well dissolved in EA and then placing the separating funnel on a stand for incubation to separate water and EA phases; and removing only the water phase after the water and EA phases were separated. The EA phase was transferred to a new RBF and then vacuum-dried. DMSO was added to the vacuum-dried sample and dissolved at a concentration of 10 mg/ml 3-acetyl-myricetin. PBS (pH 7.0) was added to the solution containing 10 mg/ml 3-acetyl-myricetin and diluted to a concentration of 1 mg/ml 3-acetyl-myricetin. The sample prepared in this way was incubated overnight at a temperature of 37°C while stirring at a speed of 200 rpm. The sample that reacted overnight was then freeze-dried. The freeze-dried sample was dissolved using DMSO. For the sample prepared in this way, RP-HPLC was performed under the conditions shown in Table 1, and only the peak fraction with retention time of 16 minutes was taken.

**[Table 1]**

| **Instrument** | Agilent 1260 infinity HPLC system |
|---|---|
| **Column** | YMC Triart C18 Column (250× 10 mm) |
| **Column oven temperature** | 25°C |
| **Injection volume** | 20 µl |
| **UV detection** | 360 nm |
| **Mobile phase** | Gradient method (30% ACN → 70% ACN) |
| **Analysis time** | 40 min |
| **Buffer** | A: D.W (0.05% TFA), B: ACN |
| **Flow rate** | 2 ml/min |

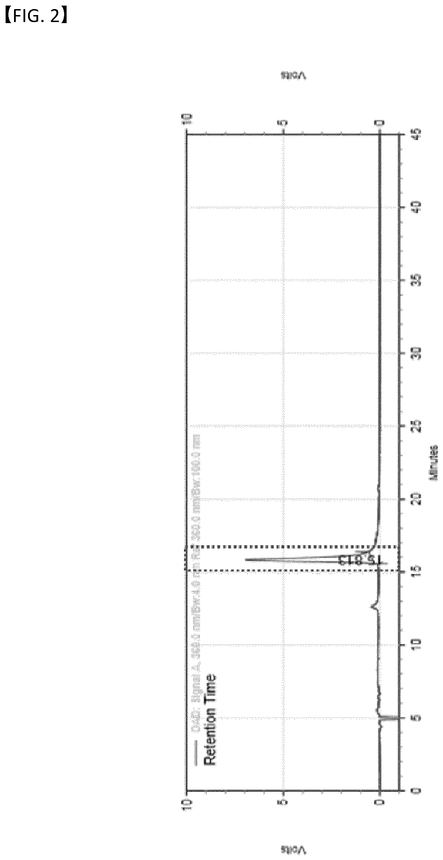

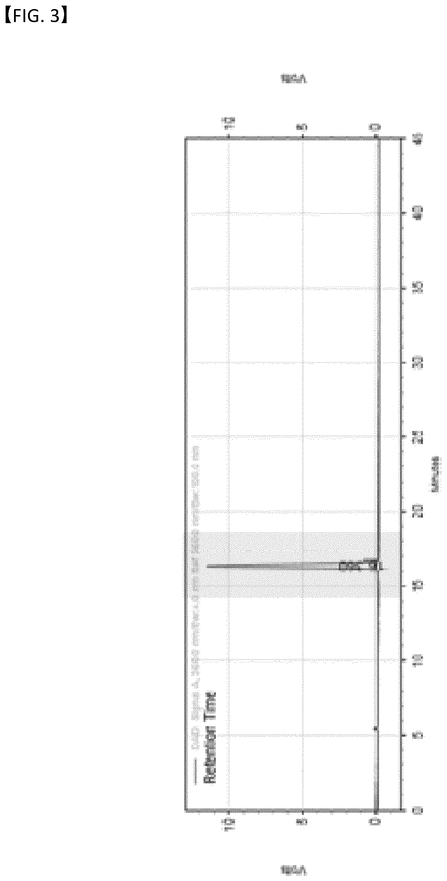

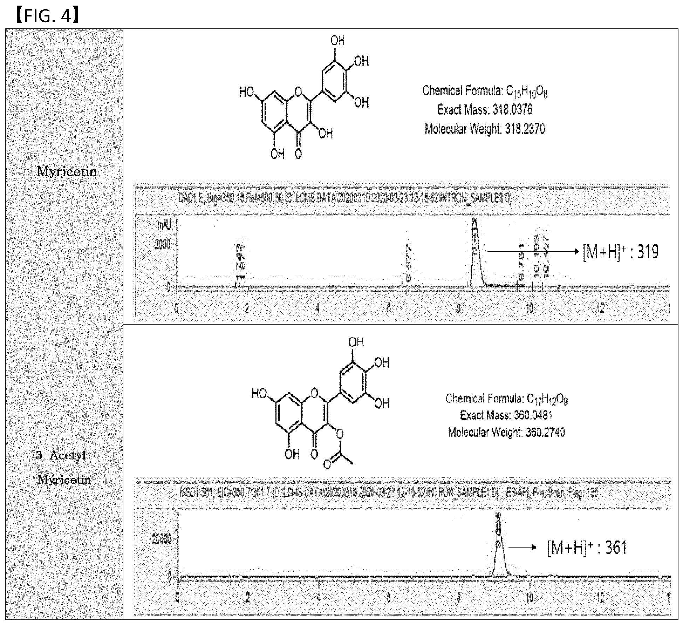

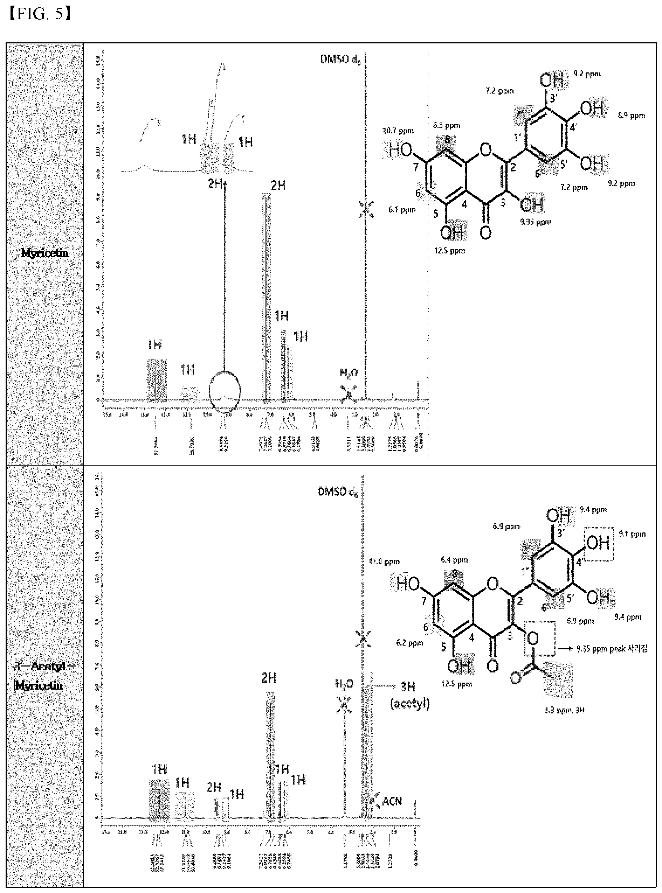

The synthesis process of 3-acetyl-myricetin represented by [Formula 1] is summarized in FIG. 1, and the RP-HPLC results are shown in FIG. 2. The results of RP-HPLC analysis for the final purified 3-acetyl-myricetin (purity of 99% or higher) represented by [Formula 1] are shown in FIG. 3. To confirm the structure, LC/MS analysis and ¹H-NMR analysis were performed according to conventional methods (During NMR analysis, the present inventors referred to the NMR analysis paper on myricetin (Molecules 2014; 19:13643)). The results are presented in FIGS. 4 and 5.

### Example 2: Investigation of neurotransmission inhibition efficacy

In this example, it was investigated whether 3-acetyl-myricetin represented by [Formula 1] prepared in Example 1 could provide the neurotransmission inhibition efficacy as expected. The investigation was performed by a conventional acetylcholine release assay which is a gold standard cell-based method determining the released acetylcholine as follows. The acetylcholine release assay is a method that quantitatively analyzes the amount of acetylcholine released from acetylcholinergic neurons..

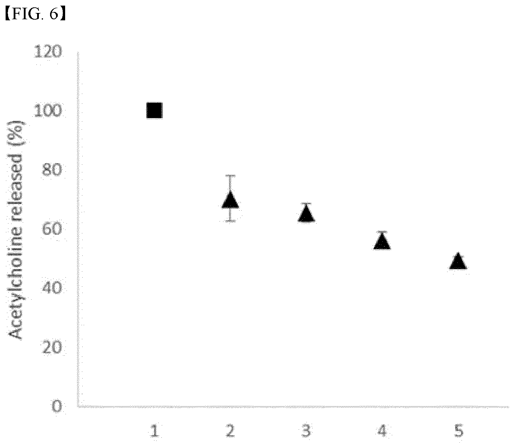

Specifically, first, PC12 cells were subcultured once every three days in a T75 flask using full serum media (84% Dulbecco's modified eagle's media (Hyclone), 10% Horse serum (Gibco), 5% Fetal bovine serum (Gibco), 1% Antibiotic antimycotic solution (Hyclone)). While subculture was not being carried out, the media were replaced with new full serum media every day. At the same time, on the first day of the experiment, a collagen-coated plate was prepared. The preparation of the collagen-coated plate was performed as follows. A 20 mM acetic acid solution was prepared using sterilized water filtered through a 0.2 µm filter. Using the acetic acid solution, 50 ml of collagen solution with a concentration of 50 ug/ml was prepared. 1 ml of the collagen solution prepared this way was added per well and then incubated at room temperature for 3 hours. After the incubation, the collagen solution from each well was carefully removed. The wells were carefully washed with 1 ml of PBS. Collagen-coated plate preparation was completed by air drying within a clean bench. On the second day of the experiment, cells were seeded onto the collagen-coated plate prepared previously. Cell inoculation was performed by the following method. The media of PC12 cells cultured in the T75 flask were removed. 2 ml of serum free media (99% Dulbecco's modified eagle's media (Hyclone), 1% antibiotic antimycotic solution (Hyclone)) was added to the flask. Afterward, the cells were removed by lightly pipetting and transferred to a 15 ml conical tube and centrifuged (2,500 rpm, 3 minutes). After centrifugation, the supernatant was removed, the cells were resuspended using 1 ml of serum free media, and cell counting was performed. A PC12 cell suspension of 2×10⁵ cells/ml was prepared using serum-free media. Afterward, 1 ml per well was dispensed into the wells of the collagen-coated plate prepared in advance. The plate was incubated (CO₂ incubator, 37°C) for 24 hours. On the third day from the start of the experiment, the media were removed from the culture plate. Next, the media were replaced with nerve growth factor (NGF) media (98.6% Dulbecco's modified eagle's media (Hyclone), 1% antibiotic antimycotic solution (Hyclone), 0.4% (25 µg/ml) nerve growth factor (NGF)-2.5S from murine submaxillary gland (Sigma)). After the NGF treatment, the media were further cultured for 5 days (CO₂ incubator, 37°C). On the 8th day of the experiment, sample processing and analysis were performed using the following method. The media were removed from the plate, and 995 µl of serum-free media were added to the plate. 5 µl of respective 0.5 µM, 2 µM, 10 µM, and 20 µM 3-acetyl-myricetin samples prepared using DMSO were added to each well and 5 µl of DMSO was added to the control well. The plate was incubated for 18 hours (CO₂ incubator, 37°C) . After 18 hours of incubation, quantitative analysis was performed using the Cell Biolabs's acetylcholine release assay kit according to the user mannual. The results are presented in FIG. 6.

From these results, it was confirmed that the myricetin derivative (3-acetyl-myricetin) represented by [Formula 1] of the present disclosure could also inhibit SNARE complex formation and consequently inhibit acetylcholine release. This shows that the myricetin derivative (3-acetyl-myricetin) represented by [Formula 1] of the present disclosure can provide a neurotransmission blocking effect.

### Example 3: Material stability comparison

In this example, a stability comparison experiment was conducted. To compare the stability under extreme conditions, the stability test was conducted under a one-day incubation condition. For reference, most of the existing myricetin or myricetin derivatives decompose and disappear when left alone for about 2 hours. The stability experiment was conducted as follows. 10 mg/ml stock solution was prepared in DMSO and the prepared stock solution was diluted to be 1 mg/ml using PBS (pH 7.0) (1/10 dilution). The test samples prepared in this way were incubated at a temperature of 37°C for 1 day while stirring at a speed of 200 rpm. After 1-day incubation, the test samples were freeze-dried and the resultant powder samples were dissolved in DMSO. The prepared HPLC analysis samples were subjected to RP-HPLC analysis under the same HPLC analysis conditions as those presented previously to compare stability. The results are presented in Table 2.

**[Table 2]**

| **Compound** | **Residual amount** |
|---|---|
| 3-Acetyl-Myricetin | ≒100% |
| Myricetin | «10% |
| Laricitrin | «10% |
| Combretol | «10% |
| Syringetin | «10% |
| Myricetin-C4 | «10% |
| Myricetin-C8 | «10% |
| Myricetin-C12 | «10% |
| Myricetin-C16 | «10% |
| Myricetin-C20 | «10% |
| Derivative E4-2 | «10% |
| Derivative E4-4 | «10% |
| Derivative E4-8 | «10% |
| Derivative E4-12 | «10% |
| Derivative E4-16 | «10% |
| Derivative E4-18 | «10% |
| Derivative E4-20 | «10% |

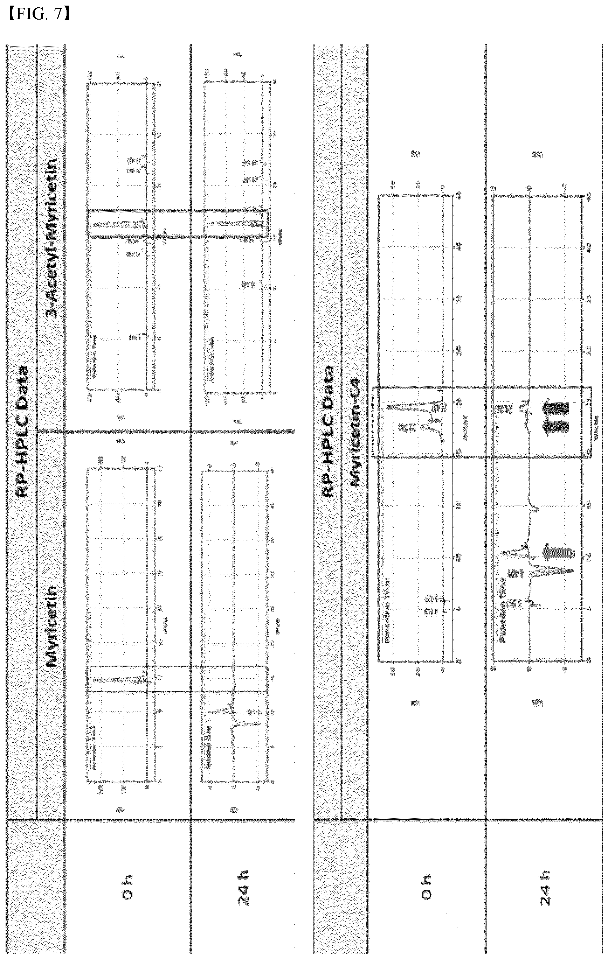

To aid understanding, representative experimental results (myricetin, myricetin-C4, and 3-acetyl-myricetin) are presented in FIG. 7.

As a result, it was confirmed that the myricetin derivative (3-acetyl-myricetin) represented by [Formula 1] of the present disclosure can provide improved stability to a degree that the previously reported myricetin derivatives cannot provide. This improved stability can serve as a great advantage in the storage and distribution of the final product prepared by applying the myricetin derivative (3-acetyl-myricetin) represented by [Formula 1]. In addition, since the experimental conditions of this example are similar to the conditions exposed in the body, the myricetin derivative (3-acetyl-myricetin) represented by [Formula 1] of the present disclosure can also provide improved in-vivo stability. Based on these results, it can also be assumed that the myricetin derivative will provide more enhanced efficacy.

### Example 4: Composition Preparation

In this example, nanoparticles entrapping various myricetin derivatives, including myricetin, were prepared. Since the efficacy comparison experiment of Example 5 was to be conducted by the method suggested in the examples of Korean Patent Application Publication No. 10-2017-0091554, nanoparticles that can be used for transdermal drug delivery were prepared. Specifically, (1) preparation of lipid phase, (2) preparation of aqueous phase, and (3) manufacturing of lipid-nanoparticles were carried out in the following order, and the detailed preparation method is as follows.

### 4.1 Preparation of lipid phase

First, a lipid phase was prepared by the following method. 70 mg of tristearin and 30 mg of tricaprylin were weighed and mixed in a glass reaction vial. Afterward, the glass reaction vial was stored in a water bath at a temperature of 85°C until the next step. 2 mg of myricetin or myricetin derivatives was prepared by dissolving in 1 ml of acetone. The myricetin or myricetin derivatives solution was added into the glass reaction vial previously prepared and the glass reaction vial was stored at 85°C for 30 minutes with stirring. After 30 minutes of reaction, vacuum drying (evaporation) was performed to remove acetone, and the lipid phase thus prepared was stored in the water bath at a temperature of 85°C.

### 4.2 Preparation of aqueous phase

To prepare an aqueous phase, 0.9735 g of Brij^{®} S 100 and 0.9735 g of Pluronic^{®} P-123 were each added into a glass reaction vessel. Afterward, 10 ml of distilled water was added to the reaction vessel, and the reaction vessel was incubated at 85°C for 12 hours with stirring.

### 4.3 Manufacturing of lipid-nanoparticles

A lipid phase (0.5 by volume) and an aqueous phase (9.5 by volume) were mixed in a 50 ml conical tube. At this time, the lipid phase and aqueous phase were required to be maintained at a temperature of 85°C. The mixture prepared this way was homogenized for 1 minute at a speed of 8,000 rpm using a highspeed blender while maintaining the temperature at 85°C and then homogenized for another minute at a speed of 10,000 rpm. Then, using a tip-type sonicator, ultrasonic mixing was performed at 60% amplitude for 4 minutes by operating for 1 second and stopping for 1 second while maintaining the temperature at 85°C. The conical tube containing the sample prepared in this way was immersed in a beaker filled with water at a temperature of 25°C and cooled to a temperature of 45°C while stirring. Afterward, ultrasonic mixing was performed at 60% amplitude for 4 minutes using a tip-type sonicator, operating for 1 second and stopping for 1 second. The prepared samples were stored in refrigeration (4°C).

### Example 5: Efficacy comparison experiment 1

An efficacy comparison experiment was performed using the composition prepared in Example 4. The efficacy comparison experiment was conducted by measuring the effect on hyperhidrosis according to the method presented in the examples of Korean Patent Application Publication No. 10-2017-0091554. Specifically, test samples were prepared by mixing the compositions prepared in Example 4 with commercially available sunscreen cream at a concentration of 1 mg/ml. The control group (untreated group) was treated with only sunscreen cream containing no composition, and the treatment groups were treated with sunscreen cream containing each composition. After applying the test samples to both hands, the subjects were asked to hold the weighed cotton pads in both hands for 5 minutes, then the cotton pads were recovered and the weight of the cotton pads was measured again to calculate the increased weight. The average values of 10 people per treatment group are presented in Table 3. The average sweat weight of the control group (untreated group) was set as 100%, and the proportional value was presented as the result.

**[Table 3]**

| **Group** | **Sweat weight** |
|---|---|
| No-treatment | 100% |
| 3-Acetyl-Myricetin-treatment | 28% |
| Myricetin-treatment | 55% |
| Laricitrin-treatment | 52% |
| Combretol-treatment | 56% |
| Syringetin-treatment | 53% |
| Myricetin-C4-treatment | 52% |
| Myricetin-C8-treatment | 55% |
| Myricetin-C12-treatment | 64% |
| Myricetin-C16-treatment | 58% |
| Myricetin-C20-treatment | 66% |
| Derivative E4-2-treatment | 53% |
| Derivative E4-4-treatment | 54% |
| Derivative E4-8-treatment | 58% |
| Derivative E4-12-treatment | 63% |
| Derivative E4-16-treatment | 65% |
| Derivative E4-18-treatment | 66% |
| Derivative E4-20-treatment | 68% |

The results show that the myricetin derivative (3-acetyl-myricetin) represented by [Formula 1] of the present disclosure can provide an improved effect to a degree that the previously reported myricetin derivatives cannot provide. This can be interpreted as being due to improved in-vivo stability.

### Example 6: Efficacy comparison experiment 2

Additional efficacy comparison experiments were conducted using mice. The composition prepared in Example 4 was also used. The specific experimental method is presented as follows. 180 6-week-old male ICR mice (body weight: 25-35 g) were randomly divided into 18 groups (10 mice per group) and then treated by skin application. Specifically, the mice were anesthetized for more than 1 hour by intraperitoneally injecting a mixed solution of 80 mg/kg Alfaxan and 10 mg/kg Rompun thereto. Then, the sample was applied to the bottom of the right hind paw of the anesthetized mice. The negative control group received application of PBS (pH 7.4) at a dose of 0.8 ml/kg. The treatment groups received the application of each composition prepared in Example 4 (prepared by suspending in PBS) at a dose of 0.8 mg/kg. To ensure that the applied sample was sufficiently absorbed, the mice to which the sample had been applied were kept as the mice were for 30 minutes. Afterward, the sample on the sole was wiped, and then 3.5% iodine solution (w/v) dissolved in ethanol was applied again. After drying for 1 minute, a 10% starch solution (w/v) dissolved in castor oil was additionally applied. Immediately after applying the starch solution, the soles of all mice were photographed under the same conditions (distance, magnification, and exposure) using a 12-megapixel digital camera. The sweating area shown in the taken photos was analyzed through image analysis. The results are presented in FIG. 8.

This result showed that the myricetin derivative (3-acetyl-myricetin) represented by [Formula 1] of the present disclosure can provide an improved effect to a degree that the previously reported myricetin derivatives cannot provide. This can also be interpreted as being due to improved in-vivo stability.

### Example 7: Efficacy comparison experiment 3

A comparative experiment was conducted on the efficacy of improving skin wrinkles. The experiment was conducted on 72 women aged 30 to 50, divided into one group of four. Before the experiment begins, replicas from under the eyes are collected using the usual method. One hour after the collection, a test sample (5 ml) of the composition prepared in Example 4 to have a concentration of 1 mg/ml using PBS was applied to the facial area for each experimental group. The facial area for the control group was applied with PBS. Six hours after the application, the replica was collected again from the same area under the eye. Dermal density of skin wrinkles was compared and measured by performing two-dimensional analysis of skin wrinkles through image analysis of the collected replicas. The measurement results of skin wrinkle density by image analysis were obtained by averaging the ratio of the dermal density of skin wrinkles after application to the dermal density of skin wrinkles before application of the test sample. The results are shown in Table 4 below.

**[Table 4]**

| **Group** | **Reduction of skin wrinkle dermal density (%)** |
|---|---|
| PBS | 5 |
| 3-Acetyl-Myricetin-treatment | 78 |
| Myricetin-treatment | 48 |
| Laricitrin-treatment | 38 |
| Combretol-treatment | 36 |
| Syringetin-treatment | 37 |
| Myricetin-C4-treatment | 52 |
| Myricetin-C8-treatment | 43 |
| Myricetin-C12-treatment | 44 |
| Myricetin-C16-treatment | 39 |
| Myricetin-C20-treatment | 40 |
| Derivative E4-2-treatment | 51 |
| Derivative E4-4-treatment | 52 |
| Derivative E4-8-treatment | 46 |
| Derivative E4-12-treatment | 47 |
| Derivative E4-16-treatment | 43 |
| Derivative E4-18-treatment | 42 |
| Derivative E4-20-treatment | 41 |

This result showed that the myricetin derivative (3-acetyl-myricetin) represented by [Formula 1] of the present disclosure can provide an improved effect to a degree that the previously reported myricetin derivatives cannot provide.

Below are examples of formulations and preparation examples for the cosmetic composition of the present disclosure. However, the following formulation examples and preparation examples are merely illustrative of the present disclosure, and the content of the present disclosure is not limited by the following formulation examples and preparation examples.

### Preparation Example 1: Preparation of flexible lotion (skin lotion)

Flexible lotion was prepared by a conventional method by mixing 0.1% (w/w) nanoparticles entrapping myricetin derivative (3-acetyl-myricetin) represented by [Formula 1], 5.2% (w/w) 1,3-butylene glycol, 1.5% (w/w) oleyl alcohol, 3.2% (w/w) ethanol, 3.2% (w/w) polysorbate 20, 2.0% (w/w) benzophenone-9, 1.0% (w/w) carboxyl vinyl polymer, 3.5% (w/w) glycerin, a trace amount of fragrance, a trace amount of preservatives, and a remaining amount of purified water.

### Preparation Example 2: Preparation of milk lotion

Milk lotion was prepared by a conventional method by mixing 0.1% (w/w) nanoparticles entrapping myricetin derivative (3-acetyl-myricetin) represented by [Formula 1], 5.1% (w/w) glycerin, 4.2% (w/w) propylene glycol, 3.0% (w/w) tocopheryl acetate, 4.6% (w/w) liquid paraffin, 1.0% (w/w) triethanolamine, 3.1% (w/w) squalane, 2.5% (w/w) macadamia nut oil, 1.6% (w/w) polysorbate 60, 1.6% (w/w) sorbitan sesquirolate, 0.6% (w/w) propylparaben, 1.5% (w/w) carboxylic vinyl polymer, a trace amount of fragrance, a trace amount of preservatives, and a remaining amount of purified water.

### Preparation Example 3: Preparation of nourishing cream

Nourishing cream was prepared by a conventional method by mixing 0.5% (w/w) nanoparticles entrapping myricetin derivative (3-acetyl-myricetin) represented by [Formula 1], 4.0% (w/w) glycerin, 3.5% (w/w) Vaseline, 2.1% (w/w) triethanolamine, 5.3% (w/w) liquid paraffin, 3.0% (w/w) squalane, 2.6% (w/w) beeswax, 5.4% (w/w) tocopheryl acetate, 3.2% (w/w) polysorbate 60, 1.0% (w/w) carboxyl vinyl polymer, 3.1% (w/w) sorbitan sesquioleate, a trace amount of fragrance, a trace amount of preservatives, and a remaining amount of purified water.

As the specific parts of the present disclosure have been described in detail above, it is clear to those skilled in the art that these specific techniques are merely preferred implementation examples and do not limit the scope of the present disclosure. Therefore, the practical scope of the present disclosure will be defined by the appended claims and their equivalents.

## Claims

1. A composition providing an effect of inhibiting acetylcholine release, the composition comprising 3-acetyl-myricetin being a myricetin derivative represented by the following [Formula 1] as an active ingredient, and

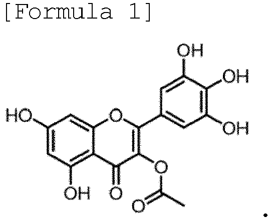

2. The composition of claim 1, wherein the composition is a cosmetic composition for aesthetic purposes or a pharmaceutical composition for treatment purposes of disorders caused by excessive release of acetylcholine.

3. The composition of claim 2, wherein the aesthetic purposes are any one selected from the group consisting of treating wrinkles, reducing pore sizes, and improving skin elasticity.

4. The composition of claim 2, wherein the disorders caused by the excessive release of acetylcholine are any one selected from the group consisting of crossed eyes, inner ear disorder, teeth grinding, keloid scarring, hyperhidrosis, back pain, tremors, anal fissure, buttock deformity, muscle injuries (twitching), chronic migraine, depression, facial nerve disorder, neck pain (spasms), thyroid disorder, cardiac muscle disorder, upper limb spasticity, pancreas disorders, irritable bowel syndrome, stretch marks, and juvenile cerebral palsy.

5. A method for preparing 3-acetyl-myricetin being a myricetin acetyl derivative represented by [Formula 1], the method comprising:
(A) performing a reaction by adding acetic anhydride to myricetin dissolved in pyridine anhydrous under a nitrogen atmosphere sealed to prevent external air and moisture from being introduced;
(B) extracting the synthesized 3-acetyl-myricetin with ethyl acetate (EA);
(C) vacuum-drying the ethyl acetate (EA) extract;
(D) dissolving by adding dimethyl sulfoxide (DMSO) to the vacuum-dried sample and then diluting with phosphate-buffered saline (PBS; pH 7.0);
(E) incubating the diluted sample to decompose byproducts; and
(F) freeze-drying the incubated sample.
